# FASCICULE DE BREVET EUROPEEN

(11) **EP 3 030 546 B1**
(45) Date de publication et mention de la délivrance du brevet: **27.12.2017**
(21) Numéro de dépôt: 14747087.6
(22) Date de dépôt: 09.07.2014
(51) Int. Cl.: C07C 407/00, C07C 409/16

(54) **PROCEDE DE PREPARATION DE PEROXYDES ORGANIQUES**
VERFAHREN ZUR HERSTELLUNG ORGANISCHER PEROXIDE
METHOD FOR PREPARING ORGANIC PEROXIDES

(30) Priorité: 08.08.2013 FR 1357893
(43) Date de publication de la demande: 15.06.2016
(73) Titulaire: Arkema France, 92700 Colombes (FR)
(72) Inventeur: MAJ, Philippe, F-69530 Brignais (FR); HUB, Serge, F-69100 Villeurbanne (FR)
(74) Mandataire: Gorintin, Sarah
(86) Numéro de dépôt international: PCT/FR2014/051764
(87) Numéro de publication internationale: WO 2015/018996

(56) Documents cités:
- US-A- 2 668 180
- US-A- 3 764 628
- US-A- 3 919 326
- DATABASE CAPLUS [Online] CHEMICAL ABSTRACTS SERVICE, COLUMBUS, OHIO, US; 1976, XP002724539, Database accession no. 1976:135300 -& SU 504 762 A1 28 février 1976 (1976-02-28) cité dans la demande

## Description

### Domaine de l'invention

L'invention a pour objet un procédé de préparation/fabrication/production de peroxydes organiques d'alkyles tertiaires par une réaction de condensation entre des composés ayant au moins un groupement alcool tertiaire et un composé ayant au moins une fonction hydropéroxyde tertiaire en présence d'un catalyseur.

La structure chimique des peroxydes est caractérisée par la présence de deux molécules d'oxygène reliées entre elles par une liaison covalente simple. Cette structure présente une instabilité propre. Les peroxydes se décomposent facilement en radicaux libres extrêmement réactifs.

Les peroxydes organiques sont très largement employés dans l'industrie chimique ainsi que dans celle des matières plastiques et celle du caoutchouc. Ils interviennent comme amorceurs dans la polymérisation radicalaire de monomères en polymères thermoplastiques, comme agents durcisseurs pour les résines polyesters thermodurcissables et comme agents de réticulation pour les élastomères et le polyéthylène. Les peroxydes organiques servent de source de radicaux libres dans de nombreuses synthèses organiques.

Le présent procédé de fabrication/production s'applique en particulier à la synthèse de peroxydes organiques de formules générales suivantes : 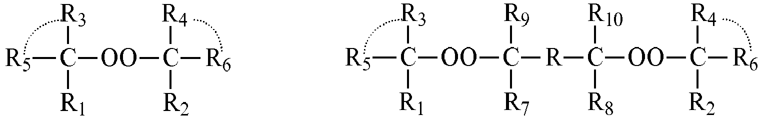 avec R₁ et R₂ sont des groupements alkyles ou aryles éventuellement substitués (alkyles, alkényles...), R₃ à R₁₀ sont des groupements alkyles contenant de 1 à 3 atomes de carbone pouvant être reliés entre eux pour former un cycle (cyclopentyl, cyclohexyl,..), R est un groupement aryle, substitué ou non, ou un groupement alkyle contenant de 1 à 4 atomes de carbone pouvant contenir des insaturations (groupements alkényles ou alkynyles).

### Etat de l'art

Une des voies de synthèse de peroxyde organique d'alkyles par catalyse acide utilisée industriellement requière de grande quantité d'agent(s) de condensation tel que l'acide sulfurique. L'utilisation de grande quantité d'acide sulfurique implique un coût de traitement des phases aqueuses élevé dû à des quantités importantes de sulfate générées dans les effluents.

Ce procédé de synthèse est divulgué par exemple dans le document GB 1049989. Une solution aqueuse d'acide sulfurique à 62,5 % est utilisée comme catalyseur et représente 49 % en poids de la préparation.

Par ailleurs, un procédé de synthèse nécessitant une concentration importante d'acide sulfurique présente un risque industriel sérieux. Plus la concentration en acide est forte, plus la température doit être rigoureusement maintenue à des températures faibles. Afin de contrôler la réaction, ce type de procédé nécessite des conditions de température généralement inférieure à 10 °C. Plus la concentration en acide est forte et plus la synthèse de peroxyde nécessite une température basse dans le milieu réactionnel, typiquement inférieure ou égale à 5 °C. Afin d'obtenir un rendement élevé, cette voie de synthèse requiert une concentration d'acide importante, mais alors les coûts liés au nécessaire refroidissement deviennent prohibitifs.

En plus des coûts liés au refroidissement, l'inconvénient d'un rendement relativement faible et de la production de quantités importantes de sulfates dans les effluents, ce procédé produit une quantité non négligeable de sous produits de type phénolique qui encrassent le réacteur. Le réacteur nécessite ainsi un nettoyage régulier, ce qui ralentit la production et représente un coût additionnel important.

Les grandes quantités d'acide nécessaires avec cette voie de synthèse trouvent leur origine en particulier dans l'eau formée lors de la réaction de condensation qui dilue très significativement l'effet catalytique des agents de condensation acide.

Une méthode alternative au procédé comprenant de haute concentration en acide sulfurique, consiste à éliminer l'eau formée, en présence d'un solvant organique, par distillation azéotropique sous pression réduite. Des quantités inférieures à 1 % d'agent de condensation acide peuvent être ainsi utilisées.

Le document US 3919326 divulgue un procédé de préparation de peroxydes organiques en présence de moins de 1 % d'acide sulfonique de para-toluène. L'eau formée lors de la réaction de condensation est éliminée au fur et a mesure de l'avancement de la réaction, avec le solvant organique, par distillation azéotropique, sous pression réduite.

Ce procédé limite la quantité de sels d'acide générés dans les effluents mais requièrent l'utilisation d'équipements spéciaux d'extraction sous vide de l'eau formée, limitant ainsi leurs utilisations.

Des procédés n'utilisant pas de quantité importante d'agent de condensation ou ne nécessitant pas des conditions d'extraction sous vide ont été proposés, tels que ceux divulgués dans les documents US 2668180 et FR 2379518.

Le document US 2668180 divulgue un procédé de production de peroxydes organiques à pression atmosphérique, à haute température (80-115 °C) en présence d'une quantité relativement faible d'agent de condensation acide comme catalyseur. Les agents de condensation acide utilisés sont l'acide sulfonique de para-toluène, l'acide sulfurique et l'éthérate de trifluorure de bore. Les rendements en peroxydes obtenus ne sont pas satisfaisants. De plus, les hautes températures utilisées peuvent induire des réactions parasites de décompositions thermiques des peroxydes.

Le document FR 2379518 divulgue un procédé d'obtention de peroxyde organique en présence de solution aqueuse d'acides minéraux tels que l'acide chlorhydrique et l'acide nitrique. Les peroxydes ainsi produits précipitent massivement dans le milieu réactionnel et sont séparés par filtration ou décantation.

Ces procédés ne donnent pas de rendements satisfaisants et l'obtention de précipités important lors de la synthèse complique de manière très importante le procédé de fabrication, ce qui induit notamment des inconvénients économiques majeurs.

On connait par ailleurs le document EP 0967194 qui divulgue un procédé d'obtention de peroxyde en présence d'acide sulfonique comme catalyseur à une température supérieure à 40 °C à pression atmosphérique. Le catalyseur est ajouté progressivement sur une période d'une heure afin de limiter toute exothermicité. Un rendement de 70 % à 80 % est obtenu au minimum au bout de deux heures de réaction.

Dans ce procédé, il faut noter que le solvant utilisé est du cumène et non un alcane. De plus, ce procédé ne prévoit pas d'ajouter un acide minéral et les réactions de condensation sont relativement lentes. Enfin, les réactions parasites, telles que la déshydratation de l'alcool ou la formation de phénol, sont conséquentes.

On connaît le brevet SU 504762, publié en 1976, qui divulgue un procédé de préparation de 1,4-bis-[2-(*tert*-butyl-peroxy)isopropyl]benzène par une réaction de condensation de *tert*-butyl hydroperoxyde et 1,4-bis-[2-hydroxyisopropyl]benzène en présence de l'acide sulfurique et d'anhydride acétique.

On connaît enfin le brevet US 3764628 qui divulgue un procédé de préparation de dérivés de bis(alkylperoxy-alkyl)benzène par réaction d'un bishydroxyalkylbenzène et d'un hydropéroxyde tertiaire en présence de l'acide sulfurique comme catalyseur.

Il existe donc un besoin important d'obtenir un procédé industriel de production de peroxyde organique ne présentant pas les inconvénients de l'art antérieur.

### Brève description de l'invention

De façon surprenante, la demanderesse a découvert, après diverses expériences et manipulations que, contrairement aux enseignements bien connus de l'homme du métier, l'utilisation simultanée de quantité d'acide sulfonique et d'acide minéraux dans des proportions déterminées, permet de mener la réaction de production de peroxyde organique dans des conditions facilement réalisable industriellement, sans avoir besoin d'éliminer l'eau formée tout au long de la réaction, tout en obtenant des rendements élevés. De plus, le procédé selon l'invention ne génère pas, ou en quantité négligeable, de sous-produits, plus particulièrement de type phénolique lorsque les réactifs sont aromatiques (au moins le composant portant la fonction alcool contient une fonction aromatique, voire le composé comportant la fonction hydropéroxyde). Ces sous-produits réduisent le rendement en produit souhaité, diminuent la pureté finale du produit et peuvent être à l'origine d'encrassement du réacteur (sous-produits phénoliques).

Ainsi, la présente invention se rapporte à un procédé de préparation de peroxyde, comprenant une étape de mise en contact dans un milieu réactionnel d'un composant ayant au moins un groupement alcool tertiaire avec un composé ayant au moins une fonction hydropéroxyde tertiaire en présence d'un catalyseur caractérisé en ce que le catalyseur comprend un acide sulfonique et un acide minéral, le rapport en moles entre l'acide sulfonique et le susdit composant comprenant au moins un groupement alcool tertiaire est compris entre 0,05 et 0,8, et le rapport en moles entre l'acide minéral et le susdit composant comprenant au moins un groupement alcool tertiaire est compris entre 0,05 et 0,8.

Aucun document de l'art antérieur ne divulgue l'utilisation d'un mélange d'acide minéral et d'acide sulfonique à pression atmosphérique pour la préparation/fabrication/production de peroxydes. De plus, de par ses connaissances générales, pour des raisons de sécurité, l'homme du métier n'est pas amené à considérer l'utilisation d'un procédé de production de peroxyde comprenant un acide minéral relativement concentré, tel que l'acide sulfurique, à des températures supérieures à la température ambiante. Le mélange d'acide minéral et d'acide sulfonique permet de travailler à des températures supérieures à l'ambiante sans engendrer de risque(s) au niveau de la sécurité.

L'invention présente notamment les avantages suivants :
- d'utiliser des conditions industrielles classiques (sans nécessité des équipements complexe et couteux), nécessitant simplement une pression atmosphérique et une température légèrement supérieure à l'ambiante.
- de ne pas nécessiter le retrait de l'eau formée tout au long de la réaction,
- d'utiliser une quantité d'acide plus faible que dans le cas de l'utilisation d'acides minéraux seuls, et de permettre ainsi, dans le cas d'acide sulfurique, de limiter la quantité de sulfate rejeté dans les effluents,
- d'obtenir un rendement en peroxyde organique élevé du fait en particulier d'un effet synergique des cinétiques par rapport à celle des acides pris séparément,
- de réduire le temps d'introduction du catalyseur par rapport au procédé utilisant une grande quantité d'acide minéral seul.

D'autres caractéristiques avantageuses de l'invention sont précisées dans la suite : Avantageusement, le rapport en moles entre l'acide sulfonique et le composant comprenant au moins un groupement alcool tertiaire est compris entre 0,1 et 0,6, préférentiellement compris entre 0,1 et 0,3.
Avantageusement, le rapport en moles entre l'acide minéral et le composant comprenant au moins un groupement alcool tertiaire est compris entre 0,1 et 0,6, préférentiellement compris entre 0,1 et 0,5.
Selon un aspect particulièrement intéressant de l'invention, le procédé est réalisé à pression atmosphérique (± 0,2 bar).
Selon un mode d'exécution préféré de l'invention, le susdit composant et/ou le susdit composé comporte une ou plusieurs fonctions aromatiques de sorte que le peroxyde comprend au moins une fonction aromatique.
Selon une possibilité offerte par l'invention, le susdit composé comportant un groupement hydropéroxyde est choisi parmi l'hydropéroxyde de tert-butyle, l'hydropéroxyde de tert-amyle, le 1-methylcyclohexylhydroperoxyde, le 1-methylcyclopentylhydroperoxyde, préférentiellement l'hydropéroxyde de tert-butyle. Selon une autre possibilité offerte par l'invention, le susdit composé comportant un groupement hydropéroxyde est choisi parmi le 2,5-diméthyle-2,5-di-hydroperoxy-3-hexyne ou le 2,5-diméthyle-2,5-dihydroperoxyhexane. Selon un mode de réalisation de l'invention, le susdit composant comportant un groupement alcool est choisi parmi le tert-butanol, tert-amyle alcool, le cumylalcool, le 1-methylcyclohexanol, le 1-methylcyclopentanol, préférentiellement le cumylalcool.
Selon un autre mode de réalisation de l'invention, le susdit composant comportant un groupement alcool est choisi parmi le α,α'-dihydroxy-diisopropylbenzène, le 2,5-dimethyl-2,5-di-hydroxy-3-hexyne, le 2,5-diméthyle-2,5-dihydroxy-hexane, et plus préférentiellement le α,α'-dihydroxy-diisopropylbenzène.

L'acide sulfonique est choisi de préférence parmi les acides sulfoniques d'alkyles ou d'aryles, plus particulièrement les acides aryles sulfoniques tels que l'acide sulfonique de benzène, l'acide sulfonique de para-toluène, l'acide sulfonique de naphtalène, l'acide sulfonique de xylène, l'acide sulfonique de cumène, et leurs mélanges, et préférentiellement choisi parmi l'acide sulfonique de cumène et l'acide sulfonique de para toluène.

Concernant l'acide minéral, il est choisi préférentiellement parmi l'acide sulfurique, l'acide chlorhydrique, l'acide nitrique, l'acide phosphorique, l'acide perchlorique, et leurs mélanges, préférentiellement choisi parmi l'acide sulfurique.

De préférence, l'excès stoechiométrique entre les réactifs, à savoir entre le susdit composé comprenant au moins un groupement hydropéroxyde tertiaire et le susdit composant contenant au moins une fonction alcool tertiaire est compris entre 0,01 et 1, préférentiellement compris entre 0,05 et 0,5, plus préférentiellement compris entre 0,1 et 0,3.

On parle ici d'excès stoechiométrique car le rapport hydropéroxyde sur alcool doit respectivement d'au moins être de 1 (mole) sur 1 (mole) pour un monoperoxyde et de 2 (moles) sur 1 (mole) pour un diperoxyde. Ainsi, par exemple dans le cas de la préparation du mélange d'isomères méta et para de l'α,α'-Bis(tert-butylperoxy)-diisopropylbenzene, on opèrera avec un excès d'hydropéroxyde par rapport au diol. Mais cet excès stoechiométrique pourra également être à l'avantage de l'alcool et non de l'hydropéroxyde, par exemple dans le cas de préparation du 2,5-Di-(1-methylcyclohexylperoxy)-2,5-diméthyle-3-hexyne.

Avantageusement, l'étape de mise en contact est réalisée à une température comprise entre 10 °C et 60 °C, préférentiellement comprise entre 20 °C et 50 °C.

Selon un aspect de l'invention, le procédé peut comprendre une étape préalable à la susdite étape de mise en contact dans le milieu réactionnel consistant en une étape de mélange de l'acide minéral avec l'acide sulfonique hors dudit milieu réactionnel.

Selon un mode de réalisation préféré de l'invention, le catalyseur consiste uniquement en l'acide sulfonique et l'acide minéral.

L'invention se rapporte également au peroxyde directement obtenu par le procédé de préparation défini ci-dessus. Un tel peroxyde présente la particularité avantageuse d'être quasiment pur à l'issue de sa préparation de sorte qu'il ne nécessite aucun lavage destiné à lui évacuer des impuretés. Dans certains cas, lorsque un niveau de pureté (absence de traces de sous-produits, en particulier des acides) est recherché, il pourra être néanmoins souhaitable de réaliser une voire deux opérations de lavage du peroxyde. Ainsi, le peroxyde obtenu par le procédé selon l'invention se distingue des peroxydes de l'art antérieur en ce que, une fois obtenu, il ne comporte qu'un taux très faible d'impureté, en particulier d'acide (susceptible d'être préjudiciable aux applications futures du peroxyde), typiquement inférieur à 50 ppm (partie par million), voire de préférence inférieure à 20 ppm.

Ainsi, le peroxyde obtenu par le procédé de préparation selon l'invention comprend moins de 50 ppm d'acide, de préférence moins de 20 ppm.

D'autres avantages apparaîtront éventuellement à la lecture de la description qui suit. La description qui va suivre est donnée uniquement à titre illustratif et non limitatif.

### Description détaillée de l'invention

L'invention concerne un procédé de production de peroxyde comprenant une étape de mise en contact d'un composé comprenant au moins un groupement alcool tertiaire avec un composé contenant au moins une fonction hydropéroxyde tertiaire en présence d'un catalyseur. Le composé comprenant au moins un groupement alcool tertiaire réagit par condensation avec un composé contenant au moins une fonction hydropéroxyde tertiaire par catalyse acide.

Les peroxydes produits selon le procédé de l'invention sont des peroxydes de dialkyle pouvant contenir jusqu'à deux fonctions peroxydes O-O (Oxygène-Oxygène).

S'agissant des composés comprenant au moins groupement alcool tertiaire, il pourra s'agir de tert-butylalcool, de tert-amylalcool, du cumylalcool, du 1-methylcyclohexanol, du 1-methylcyclopentanol, du α,α'-dihydroxy-diisopropylbenzène, du 2,5-diméthyle-3-hexyne-2,5-diol, du 2,5-diméthyle-2,5-hexanediol.

S'agissant du composé contenant au moins une fonction hydropéroxyde tertiaire il pourra s'agir de l'hydropéroxyde de tert-butyle, de l'hydropéroxyde de tert-amyle, du 1-methylcyclohexylhydroperoxyde, du 1-Methylcyclopentylhydroperoxyde, du 2,5-diméthyle-2,5-dihydroperoxy-2-hexyne, du 2,5-diméthyle-2,5-dihydroperoxyhexane.

Le procédé de production de peroxyde organique de dialkyle selon l'invention est réalisé par la mise en contact d'un alcool avec un hydropéroxyde en présence d'un catalyseur.

Le procédé selon l'invention comprend l'utilisation d'un catalyseur comprenant l'association d'un acide minéral et d'un acide sulfonique. Seuls ces deux éléments (acides minéral et sulfonique) sont nécessaires pour réaliser l'invention (résoudre les problèmes techniques), mais on pourra éventuellement envisager d'associer d'autres éléments pour constituer le catalyseur.

S'agissant des acides minéraux, il pourra s'agir de l'acide sulfurique, de l'acide chlorhydrique, de l'acide perchlorique, de l'acide nitrique, de l'acide phosphorique et de leurs mélanges.

S'agissant des acides sulfoniques, il pourra s'agir des acides sulfoniques d'alkyles comme l'acide méthane sulfonique, des acides sulfoniques d'alkyles fluorés comme l'acide trifluorométhanesulfonique, des acides aromatiques sulfoniques, comme l'acide sulfonique de benzène, l'acide sulfonique de para-toluène, l'acide sulfonique de para-phénol, l'acide sulfonique de naphtalène, l'acide sulfonique de xylène, l'acide sulfonique de cumène, et de leurs mélanges.

L'acide minéral et l'acide sulfonique, selon le procédé de l'invention, peuvent être ajoutés séparément ou être préalablement mélangés avant introduction dans le milieu réactionnel.

Le procédé selon l'invention est réalisé de préférence en présence de solvant organique. A titre de solvant, on peut citer, le pentane, l'hexane, l'heptane, le benzène, le toluène, les xylènes, le cumène, les hydrocarbures chlorés.

| Peroxyde | formule | Hydropéroxyde | formule | Alcool | formule |
|---|---|---|---|---|---|
| Peroxyde de di-tertbutyle | 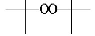 | Hydropéroxyde de tert-butyle | 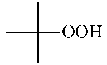 | Tertiobutanol | 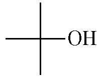 |
| Peroxyde de di-tertamyle | 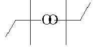 | Hydropéroxyde de tert-amyle | 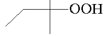 | Tertamylalcool | 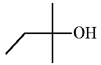 |
| Peroxyde de tert-butyle cumyle | 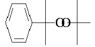 | Hydropéroxyde de tert-butyle | 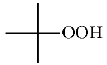 | Cumylalcool | 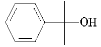 |
| Peroxyde de dicumyle | 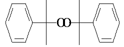 | Hydropéroxyde de cumyle | 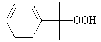 | Cumylalcool | 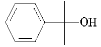 |
| Peroxyde de tert-amyle cumyle | 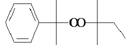 | Hydropéroxyde de tert-amyle | 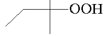 | Cumylalcool | 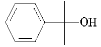 |
| Peroxyde de bis-(1-methylcyclo pentyle) | 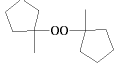 | Hydropéroxyde de 1-methyl-cyclopentyle | 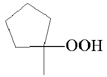 | 1-methylcyclopentanol | 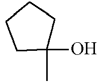 |
| α,α`-bis-(tertiobutyl peroxy)di-isopropylbe nzène | 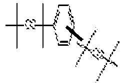 | Hydropéroxyde de tert-butyle | 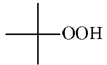 | α,α'-dihydroxy-diisopropylbenz ène mélange d'isomères ou non | 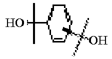 |
| 2,5-Di(tert-butylperoxy )-2,5-dimethylhe xane | 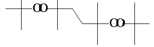 | 2,5-diméthyle-2,5-dihydro-peroxy-hexane | 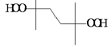 | Tertiobutanol | 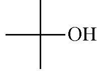 |
| 2,5-Di(tert-butylperoxy )-2,5-dimthyl-3-hexyne | 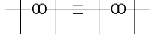 | 2,5-diméthyle-2,5-dihydro-peroxy-hexyne-3 | 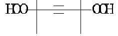 | Tertiobutanol | 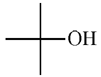 |
| 2,5-Di-(1-methylcyclo pentylperox y)-2,5-diméthyle-3-hexyne | 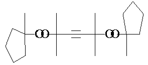 | 2,5-diméthyle-2,5-dihydro-peroxy-hexyne-3 | 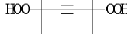 | 1 -methylcyclopentanol | 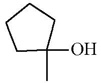 |

Le procédé selon l'invention ne requière pas d'éliminer l'eau formée par la réaction de condensation au fur et à mesure de l'avancement de la réaction de production de peroxyde, de manière azéotropique ou autre.

Une fois le peroxyde obtenu, il est classiquement envisagé au moins une étape finale d'élimination de la phase aqueuse après décantation, ainsi que des étapes bien connues de l'homme du métier, telles que des étapes de neutralisation basique du catalyseur, lavages aqueux, élimination du solvant de réaction et de purification.

Dans la suite sont présentés des exemples de réalisation du procédé de préparation selon l'invention. Ces exemples sont illustrés avec l'obtention du mélange d'isomères méta et para de l'α,α'-Bis(tert-butylperoxy)-diisopropylbenzene.

Mais des expérimentations ont été menées sur tous les peroxydes listés dans ce tableau.

Il ressort que l'obtention des peroxydes compostant au moins un noyau aromatique est particulièrement adaptée au procédé de préparation selon l'invention (l'un des réactifs au moins, classiquement le composant portant la fonction alcool, comportant un noyau aromatique).

Néanmoins, l'obtention des peroxydes aliphatiques, incluant les peroxydes comportant des cycles saturés, est également avantageusement réalisé par le procédé de préparation selon l'invention et permet de résoudre la quasi-totalité, voire la totalité, des problèmes techniques rencontrés avec les procédés de préparation de l'art antérieur.

### Exemple 1 (selon l'invention) : mise en oeuvre du procédé de synthèse selon l'invention

Dans un réacteur de 250 millilitres (ml) muni d'une vanne de fond, d'un agitateur, d'une sonde de température et d'un réfrigérant à reflux, on introduit 66,5 grammes (g) d'une solution d'hydropéroxyde de tertiobutyle (TBHP) 40,5 wt% (l'expression « wt% » signifie « weight percent » ou « pourcentage en poids ») dans de l'heptane et 27,8 g d'un mélange d'isomères (méta/para) de α,α`-dihydroxy-diisopropylbenzène à 93 wt% (diol). On chauffe le mélange à 30°C tout en l'agitant sous azote. A cette température, on ajoute 8,6 g d'une solution d'acide cumylsulfonique (65 wt%) en une seule injection. Puis on ajoute graduellement 8 grammes (g) d'une solution d'acide sulfurique (70 wt%) en environ deux (2) minutes. La température du milieu s'élève à 37-40°C. Quand l'addition des acides est achevée, la température est maintenue à 40°C pendant une durée de 115 min (minutes) tout en agitant le milieu.

Après cette période, on arrête l'agitation tout en maintenant le réacteur à 40°C. Deux phases décantent. On élimine la phase inférieure (aqueuse - 25,6 g) du réacteur par la vanne de fond.

En reprenant l'agitation, la phase supérieure (organique) est lavée à 40 °C avec 63,2 g d'eau. Après cinq (5) minutes de lavage l'agitation est stoppée pour séparer deux phases. On élimine la phase inférieure (aqueuse - 63,8 g) par la vanne de fond.

En reprenant l'agitation, la phase supérieure (organique) est lavée à 40°C avec 62,4 g d'une solution de soude (15 wt%). Après 5 minutes de lavage, l'agitation est stoppée pour séparer deux phases. On élimine la phase inférieure (aqueuse - 64.2g) par la vanne de fond.

On termine par un dernier lavage à 40°C de la phase supérieure (organique) avec 60,7 g d'eau. Après 5 min de lavage l'agitation est stoppée pour séparer deux phases. On récupère une phase inférieure (aqueuse : 61,2 g) et une phase supérieure (organique : 77,3 g).

Après analyse, la solution organique contient 41,2g de α,α`-bis-(tertiobutylperoxy) diisopropylbenzène (C₂₀H₃₄O₄), 0,35g de α-(tertiobutylperoxy)-α`-hydroxy-isopropylbenzène (C₁₆H₂₆O₃), et 0,43g de α-(tertiobutylperoxy)-α'-isopropènylbenzène (C₁₆H₂₄O₂) correspondant respectivement à la somme des isomères méta et para de chacun des composés. Ce qui représente un rendement en α,α'-bis-(tertiobutylperoxy) diisopropylbenzène de 92% par rapport au diol engagé. Le bilan matière sur ces trois produits aromatiques est de 93% par rapport au diol engagé.

### Exemple 1 bis

On procède de la même manière que dans l'exemple n°1 mais en utilisant comme alcool tertiaire du cumylalcool et les conditions opératoires décrites dans le tableau suivant.

| **Exemple** | | **1 bis** |
|---|---|---|
| Acide sulfonique | | CUSA |
| Température | °C | 30 |
| Durée de réaction | min | 30 |
| Ratio TBHP/cumylalcool | mol | 1.2 |
| [acide sulfonique] | Poids % | 65 |
| [H₂SO₄] | Poids % | 70 |
| Ratio acide sulfonique/cumylalcool | mol | 0,1 |
| Ratio H₂SO₄/diol | mol | 0,19 |
| Bilan aromatiques | mol% | 97 |
| Rendement peroxyde de tert-butylecumyle | mol% | 96.6 |

### Exemple 1 ter

On procède de la même manière que dans l'exemple n°1 mais en utilisant comme alcool tertiaire du cumylalcool, comme hydroperoxyde tertiaire de l'hydroperoxyde de cumyle, comme solvant du cumène et les conditions opératoires décrites dans le tableau suivant.

| **Exemple** | | **1 ter** |
|---|---|---|
| Acide sulfonique | | CUSA |
| Température | °C | 30 |
| Durée de réaction | Min | 30 |
| Ratio hydroperoxyde de cumyle/cumylalcool | Mol | 1.2 |
| [acide sulfonique] | Poids % | 65 |
| [H₂SO₄] | Poids % | 70 |
| Ratio acide sulfonique/cumylalcool | mol | 0,1 |
| Ratio H₂SO₄/cumyl alcool | mol | 0,16 |
| Bilan aromatiques | mol% | 95 |
| Rendement peroxyde de di-cumyle | mol% | 82 |

### Exemple 1 quatro

On procède de la même manière que dans l'exemple n°1 mais en utilisant comme alcool tertiaire du tertamylalcool, comme hydroperoxyde tertiaire de l'hydroperoxyde de tert-amyle, de l'acide methanesulfonique (MSA) et les conditions opératoires mentionnées dans le tableau suivant.

On notera ici que cet exemple 1 quatro répond à la définition de la revendication principale (revendication 1) mais pas aux revendications dépendantes 2 et 3.

| **Exemple** | | **1 quatro** |
|---|---|---|
| Acide sulfonique | | MSA |
| Température | °C | 30 |
| Durée de réaction | Min | 120 |
| Ratio hydroperoxyde de tert-amyle/tertamylalcool | Mol | 1.1 |
| [acide sulfonique] | Poids % | 68 |
| [H₂SO₄] | Poids % | 70 |
| Ratio acide sulfonique/tertamylalcool | mol | 0,37 |
| Ratio H₂SO₄/tertamylalcool | mol | 0,75 |
| Rendement peroxyde de di-tertamyle | mol% | 75 |

### Contre-exemples 2 et 3.

On procède de la même manière que dans l'exemple n°1 mais en utilisant chacun des deux acides séparément.

| **Contre-exemples** | | **2** | **3** |
|---|---|---|---|
| Acide | | CUSA | H₂SO₄ |
| Température | °C | 40 | 40 |
| Durée de réaction | min | 120 | 120 |
| Ratio TBHP/diol | mol | 2,2 | 2.3 |
| [acide] | Poids % | 65 | 70 |
| Ratio acide /diol | mol | 0,21 | 0,32 |
| Bilan aromatiques | mol | 97 | 86 |
| Rdt C₂₀H₃₄O₄ | mol | 58,3 | 8,4 |

On constate que l'utilisation d'un acide seul, qu'il soit sulfonique ou minéral, dans les proportions décrites dans l'exemple 1, ne permet pas d'atteindre le même rendement en peroxyde souhaité et reste bien inférieure à celui obtenu en associant les deux acides.

L'association des deux acides conduit également à un meilleur résultat (en termes de rendement) que la somme des résultats obtenus en utilisant chaque acide isolément, reflétant ainsi un effet de synergie des deux acides.

On peut noter que le bilan matière des entités aromatiques se dégrade en utilisant l'acide sulfurique seul dans les conditions mentionnées. Ce phénomène n'est pas observé en associant un acide sulfonique, ce qui constitue aussi un des avantages de cette invention.

### Contre-exemples 2 bis et 3 bis.

On procède de la même manière que dans l'exemple n°1 bis mais en utilisant chacun des deux acides séparément.

| **Contre-exemples** | | **2 bis** | **3 bis** |
|---|---|---|---|
| Acide | | CUSA | H₂SO₄ |
| Température | °C | 30 | 30 |
| Durée de réaction | min | 30 | 30 |
| Ratio TBHP/cumylalcool | mol | 1,2 | 1.2 |
| [acide] | Poids % | 65 | 70 |
| Ratio acide/cumylalcool | mol | 0,1 | 0,19 |
| Bilan aromatiques | Mol% | 98 | 86 |
| Rdt peroxyde de tert-butylecumyle | Mol% | 34,4 | 41.3 |

### Contre-exemples 2 ter et 3 ter.

On procède de la même manière que dans l'exemple n°1 ter mais en utilisant chacun des deux acides séparément.

| **Contre-exemples** | | **2 ter** | **3 ter** |
|---|---|---|---|
| Acide | | CUSA | H₂SO₄ |
| Température | °C | 30 | 30 |
| Durée de réaction | min | 30 | 30 |
| Ratio hydroperoxyde de cumyle/cumylalcool | mol | 1,2 | 1.2 |
| [acide] | Poids % | 65 | 70 |
| Ratio acide/cumylalcool | mol | 0,1 | 0,2 |
| Bilan aromatiques | Mol% | 95 | 87 |
| Rdt peroxyde de dicumyle | Mol% | 32 | 31 |

### Contre-exemples 2 quatro et 3 quatro

On procède de la même manière que dans l'exemple n°1 quatro mais en utilisant chacun des deux acides séparément.

| **Contre-exemples** | | **2 quatro** | **3 quatro** |
|---|---|---|---|
| Acide | | MSA | H₂SO₄ |
| Température | °C | 30 | 30 |
| Durée de réaction | min | 120 | 120 |
| Ratio Hydroperoxyde de tert-amyle/tertamylalcool | mol | 1,2 | 1.2 |
| [acide] | Poids % | 68 | 68 |
| Ratio acide/tertamylalcool | mol | 0,39 | 0,73 |
| Rdt peroxyde de di-tertamyle | Mol% | 3.7 | 23.5 |

### Exemples 4 à 6 (selon l'invention).

On procède de la même manière que dans l'exemple n°1 mais en changeant la nature de l'acide sulfonique utilisé : l'acide sulfonique de para-toluène (PTSA), l'acide sulfonique de benzène (BSA), l'acide sulfonique de phénol (PPSA).

| **Exemple** | | **4** | **5** | **6** |
|---|---|---|---|---|
| Acide sulfonique | | PTSA | BSA | PPSA |
| Température | °C | 40 | 40 | 40 |
| Durée de réaction | min | 120 | 120 | 120 |
| Ratio TBHP/diol | mol | 2.2 | 2,3 | 2,3 |
| [acide sulfonique] | Poids % | 70 | 70 | 65 |
| [H₂SO₄] | Poids % | 70 | 70 | 70 |
| Ratio acide sulfonique/diol | mol | 0,209 | 0,209 | 0,213 |
| Ratio H₂SO₄/diol | mol | 0,498 | 0,433 | 0,433 |
| Bilan aromatiques | mol% | 97,7 | 95,4 | 100,3 |
| Rendement C₂₀H₃₄O₄ | mol | 89,3 | 87,5 | 76,9 |

L'utilisation du mélange d'acide sulfonique avec l'acide sulfurique selon l'invention permet d'atteindre des rendements en α,α`-bis-(tertiobutylperoxy) diisopropylbenzène supérieures ou égales à 75% en, au plus, deux heures de réaction.

### Contre-exemple 7.

On procède de la même manière que dans l'exemple précédent mais en utilisant le PTSA seul, sans association avec l'acide sulfurique.

| **Contre-exemples** | | **7** |
|---|---|---|
| Acide | | PTSA |
| Température | °C | 40 |
| Durée de réaction | min | 120 |
| Ratio TBHP/diol | mol | 2.2 |
| [acide] | Poids % | 65 |
| Ratio acide /diol | mol | 0.21 |
| Bilan aromatiques | mol | 98 |
| Rdt C₂₀H₃₄O₄ | mol | 41.7 |

On constate, que l'utilisation de l'acide sulfonique seul, dans les proportions décrites, ne conduit pas à un rendement satisfaisant (< 75%).

### Exemples 8 à 16 (selon l'invention).

On procède de la même manière que dans l'exemple n°1 mais en changeant les paramètres de réaction (ratios, concentrations).

| **Exemple** | | **8** | **9** | **10** | **11** | **12** | **13** | **14** | **15** | **16** |
|---|---|---|---|---|---|---|---|---|---|---|
| Acide sulfonique | | CUSA | CUSA | CUSA | CUSA | CUSA | CUSA | CUSA | CUSA | CUSA |
| Température | °C | 40 | 40 | 40 | 40 | 40 | 40 | 40 | 30 | 50 |
| Durée de réaction | min | 94 | 90 | 111 | 120 | 150 | 120 | 90 | 120 | 129 |
| Ratio TBHP/diol | mol | 2,2 | 2,4 | 2,2 | 2,2 | 2,4 | 2,2 | 2,2 | 2,4 | 2,4 |
| [acide sulfonique] | poids % | 65 | 65 | 65 | 65 | 65 | 65 | 65 | 65 | 65 |
| [H₂SO₄] | poids % | 96 | 96 | 80 | 70 | 70 | 70 | 70 | 96 | 96 |
| Ratio Acide sulfurique/diol | mol | 0,129 | 0,17 | 0,127 | 0,21 | 0,126 | 0,17 | 0,128 | 0,169 | 0,137 |
| Ratio H₂SO₄/diol | mol | 0,345 | 0,346 | 0,344 | 0,428 | 0,348 | 0,345 | 0,346 | 0,345 | 0,136 |
| Bilan aromatiques | mol | 97.6 | 97.2 | 94.8 | 98.3 | 101 | 95.8 | 90.3 | 96.9 | 90.8 |
| Rendement C₂₀H₃₄O₄ | mol | 87.4 | 89.7 | 85.6 | 91.6 | 93.6 | 88.9 | 84.3 | 89.4 | 59.8 |

On retrouve dans tous les essais réalisés des rendements élevés en peroxyde souhaité.

L'effet synergique est retrouvé en comparant les résultats obtenus avec ceux des contrexemples 17 à 19 réalisés avec un seul acide.

| **Contre-exemples** | | **17** | **18** | **19** |
|---|---|---|---|---|
| Acide sulfonique | | CUSA | CUSA | CUSA |
| Température | °C | 40 | 40 | 40 |
| Durée de réaction | min | 120 | 120 | 120 |
| Ratio TBHP/diol | mol | 2,2 | 2,4 | 2,2 |
| [acide sulfonique] | Poids % | 65 | 65 | 65 |
| Ratio Acide sulfonique / diol | mol | 0,13 | 0,17 | 0,21 |
| Bilan aromatiques | mol | | | |
| Rdt C₂₀H₃₄O₄ | mol | 41,8 | 50,7 | 58,3 |

## Revendications

1. Procédé de préparation de peroxyde, comprenant une étape de mise en contact dans un milieu réactionnel d'un composant ayant au moins un groupement alcool tertiaire avec un composé ayant au moins une fonction hydropéroxyde tertiaire en présence d'un catalyseur **caractérisé en ce que** le catalyseur comprend un acide sulfonique et un acide minéral, le rapport en moles entre l'acide sulfonique et le susdit composant comprenant au moins un groupement alcool tertiaire est compris entre 0,05 et 0,8, et le rapport en moles entre l'acide minéral et le susdit composant comprenant au moins un groupement alcool tertiaire est compris entre 0,05 et 0,8.

2. Procédé selon la revendication 1, **caractérisé en ce que** le rapport en moles entre l'acide sulfonique et le composant comprenant au moins un groupement alcool tertiaire est compris entre 0,1 et 0,6, préférentiellement compris entre 0,1 et 0,3.

3. Procédé selon la revendication 1 ou 2, **caractérisé en ce que** le rapport en moles entre l'acide minéral et le composant comprenant au moins un groupement alcool tertiaire est compris entre 0,1 et 0,6, préférentiellement compris entre 0,1 et 0,5.

4. Procédé selon l'une quelconque des revendications 1, 2 ou 3, **caractérisé en ce que** le procédé est réalisé à pression atmosphérique (± 0,2 bar).

5. Procédé selon l'une quelconque des revendications précédentes, **caractérisé en ce que** le susdit composant et/ou le susdit composé comporte une ou plusieurs fonctions aromatiques de sorte que le peroxyde comprend au moins une fonction aromatique.

6. Procédé selon l'une quelconque des revendications précédentes, **caractérisé en ce que** le susdit composé comportant un groupement hydropéroxyde est choisi parmi l'hydropéroxyde de tert-butyle, l'hydropéroxyde de tert-amyle, le 1-methylcyclohexylhydroperoxyde, le 1-methylcyclopentylhydroperoxyde, préférentiellement l'hydropéroxyde de tert-butyle.

7. Procédé selon l'une quelconque des revendications 1 à 5, **caractérisé en ce que** le susdit composé comportant un groupement hydropéroxyde est choisi parmi le 2,5-diméthyle-2,5-di-hydroperoxy-3-hexyne ou le 2,5-diméthyle-2,5-dihydroperoxy-hexane.

8. Procédé selon l'une quelconque des revendications précédentes, **caractérisé en ce que** le susdit composant comportant un groupement alcool est choisi parmi le tert-butanol, tert-amyle alcool, le cumylalcool, le 1-methylcyclohexanol, le 1-methylcyclopentanol, préférentiellement le cumylalcool.

9. Procédé selon l'une quelconque des revendications 1 à 7, **caractérisé en ce que** le susdit composant comportant un groupement alcool est choisi parmi le α,α'-dihydroxy-diisopropylbenzène, le 2,5-diméthyle-2,5-di-hydroxy-3-hexyne, le 2,5-diméthyle-2,5-dihydroxy-hexane, et plus préférentiellement le α,α'-dihydroxy-diisopropylbenzène.

10. Procédé selon l'une quelconque des revendications précédentes, **caractérisé en ce que** l'acide sulfonique est choisi parmi les acides sulfoniques d'alkyles ou d'aryles, plus particulièrement les acides aryles sulfoniques tels que l'acide sulfonique de benzène, l'acide sulfonique de para-toluène, l'acide sulfonique de naphtalène, l'acide sulfonique de xylène, l'acide sulfonique de cumène, et leurs mélanges, et préférentiellement choisi parmi l'acide sulfonique de cumène et l'acide sulfonique de para toluène.

11. Procédé selon l'une quelconque des revendications précédentes, **caractérisé en ce que** l'acide minéral est choisi parmi l'acide sulfurique, l'acide chlorhydrique, l'acide nitrique, l'acide phosphorique, l'acide perchlorique, et leurs mélanges, préférentiellement choisi parmi l'acide sulfurique.

12. Procédé selon l'une quelconque des revendications précédentes, **caractérisé en ce que** l'excès stoechiométrique entre les réactifs, à savoir entre le susdit composé comprenant au moins un groupement hydropéroxyde tertiaire et le susdit composant contenant au moins une fonction alcool tertiaire, est compris entre 0,01 et 1, préférentiellement compris entre 0,05 et 0,5, plus préférentiellement compris entre 0,1 et 0,3.

13. Procédé selon l'une quelconque des revendications précédentes, **caractérisé en ce que** l'étape de mise en contact est réalisée à une température comprise entre 10 °C et 60 °C, préférentiellement comprise entre 20 °C et 50 °C.

14. Procédé selon l'une quelconque des revendications précédentes **caractérisé en ce qu'**il comprend une étape préalable à la susdite étape de mise en contact dans le milieu réactionnel consistant en une étape de mélange de l'acide minéral avec l'acide sulfonique hors dudit milieu réactionnel.

15. Procédé selon l'une quelconque des revendications précédentes, **caractérisé en ce que** le catalyseur consiste uniquement en l'acide sulfonique et l'acide minéral.

## Patentansprüche

1. Verfahren zur Herstellung von Peroxid, umfassend einen Schritt des Inberührungbringens einer Komponente mit mindestens einer tertiären Alkoholgruppe mit einer Verbindung mit mindestens einer tertiären Hydroperoxidfunktion in einem Reaktionsmedium in Gegenwart eines Katalysators, **dadurch gekennzeichnet, dass** der Katalysator eine Sulfonsäure und eine Mineralsäure umfasst, das Molverhältnis zwischen der Sulfonsäure und der Komponente mit mindestens einer tertiären Alkoholgruppe zwischen 0,05 und 0,8 liegt und das Molverhältnis zwischen der Mineralsäure und der Komponente mit mindestens einer tertiären Alkoholgruppe zwischen 0,05 und 0,8 liegt.

2. Verfahren nach Anspruch 1, **dadurch gekennzeichnet, dass** das Molverhältnis zwischen der Sulfonsäure und der Komponente mit mindestens einer tertiären Alkoholgruppe zwischen 0,1 und 0,6, vorzugsweise zwischen 0,1 und 0,3, liegt.

3. Verfahren nach Anspruch 1 oder 2, **dadurch gekennzeichnet, dass** das Molverhältnis zwischen der Mineralsäure und der Komponente mit mindestens einer tertiären Alkoholgruppe zwischen 0,1 und 0,6, vorzugsweise zwischen 0,1 und 0,5, liegt.

4. Verfahren nach einem der Ansprüche 1, 2 oder 3, **dadurch gekennzeichnet, dass** das Verfahren bei Normaldruck (± 0,2 bar) durchgeführt wird.

5. Verfahren nach einem der vorhergehenden Ansprüche, **dadurch gekennzeichnet, dass** die Komponente und/oder die Verbindung eine oder mehrere aromatische Funktionen enthält, so dass das Peroxid mindestens eine aromatische Funktion umfasst.

6. Verfahren nach einem der vorhergehenden Ansprüche, **dadurch gekennzeichnet, dass** die Verbindung mit einer Hydroperoxidgruppe aus tert-Butylhydroperoxid, tert-Amylhydroperoxid, 1-Methylcyclohexylhydroperoxid und 1-Methylcyclopentylhydroperoxid, vorzugsweise tert-Butylhydroperoxid, ausgewählt wird.

7. Verfahren nach einem der Ansprüche 1 bis 5, **dadurch gekennzeichnet, dass** die Verbindung mit einer Hydroperoxidgruppe aus 2,5-Dimethyl-2,5-dihydroperoxy-3-hexin und 2,5-Dimethyl-2,5-dihydroperoxyhexan ausgewählt wird.

8. Verfahren nach einem der vorhergehenden Ansprüche, **dadurch gekennzeichnet, dass** die Komponente mit einer Alkoholgruppe aus tert-Butanol, tert-Amylalkohol, Cumylalkohol, 1-Methylcyclohexanol und 1-Methylcyclopentanol, vorzugsweise Cumylalkohol, ausgewählt wird.

9. Verfahren nach einem der Ansprüche 1 bis 7, **dadurch gekennzeichnet, dass** die Komponente mit einer Alkoholgruppe aus α,α'-Dihydroxydiisopropylbenzol, 2,5-Dimethyl-2,5-dihydroxy-3-hexin und 2,5-Dimethyl-2,5-dihydroxyhexan und weiter bevorzugt α,α'-Dihydroxydiisopropylbenzol ausgewählt wird.

10. Verfahren nach einem der vorhergehenden Ansprüche, **dadurch gekennzeichnet, dass** die Sulfonsäure aus Alkyl- oder Arylsulfonsäuren, spezieller Arylsulfonsäuren wie Benzolsulfonsäure, para-Toluolsulfonsäure, Naphthalinsulfonsäure, Xylolsulfonsäure und Cumolsulfonsäure, und Mischungen davon und vorzugsweise aus Cumolsulfonsäure und para-Toluolsulfonsäure ausgewählt wird.

11. Verfahren nach einem der vorhergehenden Ansprüche, **dadurch gekennzeichnet, dass** die Mineralsäure aus Schwefelsäure, Salzsäure, Salpetersäure, Phosphorsäure, Perchlorsäure und Mischungen davon, vorzugsweise aus Schwefelsäure, ausgewählt wird.

12. Verfahren nach einem der vorhergehenden Ansprüche, **dadurch gekennzeichnet, dass** der stöchiometrische Überschuss zwischen den Reagenzien, nämlich zwischen der Verbindung mit mindestens einer tertiären Hydroperoxidgruppe und der Komponente mit mindestens einer tertiären Alkoholfunktion, zwischen 0,01 und 1, vorzugsweise zwischen 0,05 und 0,5, weiter bevorzugt zwischen 0,1 und 0,3, liegt.

13. Verfahren nach einem der vorhergehenden Ansprüche, **dadurch gekennzeichnet, dass** der Schritt des Inberührungbringens bei einer Temperatur zwischen 10 °C und 60 °C, vorzugsweise zwischen 20 °C und 50 °C, durchgeführt wird.

14. Verfahren nach einem der vorhergehenden Ansprüche, **dadurch gekennzeichnet, dass** es einen dem Schritt des Inberührungbringens in dem Reaktionsmedium vorgeschalteten Schritt umfasst, der aus einem Schritt des Mischens der Mineralsäure mit der Sulfonsäure außerhalb des Reaktionsmediums besteht.

15. Verfahren nach einem der vorhergehenden Ansprüche, **dadurch gekennzeichnet, dass** der Katalysator einzig und allein aus der Sulfonsäure und der Mineralsäure besteht.

## Claims

1. A process for preparing peroxide, comprising a step of placing in contact in a reaction medium a component containing at least one tertiary alcohol group with a compound containing at least one tertiary hydroperoxide function in the presence of a catalyst, **characterized in that** the catalyst comprises a sulfonic acid and a mineral acid, the mole ratio between the sulfonic acid and said component comprising at least one tertiary alcohol group is between 0.05 and 0.8, and the mole ratio between the mineral acid and said component comprising at least one tertiary alcohol group is between 0.05 and 0.8.

2. The process as claimed in claim 1, **characterized in that** the mole ratio between the sulfonic acid and the component comprising at least one tertiary alcohol group is between 0.1 and 0.6, preferably between 0.1 and 0.3.

3. The process as claimed in claim 1 or 2, **characterized in that** the mole ratio between the mineral acid and the component comprising at least one tertiary alcohol group is between 0.1 and 0.6, preferably between 0.1 and 0.5.

4. The process as claimed in any one of claims 1, 2 and 3, **characterized in that** the process is performed at atmospheric pressure (± 0.2 bar).

5. The process as claimed in any one of the preceding claims, **characterized in that** said component and/or said compound comprises one or more aromatic functions such that the peroxide comprises at least one aromatic function.

6. The process as claimed in any one of the preceding claims, **characterized in that** said compound comprising a hydroperoxide group is chosen from tert-butyl hydroperoxide, tert-amyl hydroperoxide, 1-methylcyclohexyl hydroperoxide and 1-methylcyclopentyl hydroperoxide, preferably tert-butyl hydroperoxide.

7. The process as claimed in any one of claims 1 to 5, **characterized in that** said compound comprising a hydroperoxide group is chosen from 2,5-dimethyl-2,5-dihydroperoxy-3-hexyne and 2,5-dimethyl-2,5-dihydroperoxyhexane.

8. The process as claimed in any one of the preceding claims, **characterized in that** said component comprising an alcohol group is chosen from tert-butanol, tert-amyl alcohol, cumyl alcohol, 1-methylcyclohexanol and 1-methylcyclopentanol, preferably cumyl alcohol.

9. The process as claimed in any one of claims 1 to 7, **characterized in that** said component comprising an alcohol group is chosen from α,α'-dihydroxydiisopropylbenzene, 2,5-dimethyl-2,5-dihydroxy-3-hexyne and 2,5-dimethyl-2,5-dihydroxyhexane, and more preferably α,α`-dihydroxydiisopropylbenzene.

10. The process as claimed in any one of the preceding claims, **characterized in that** the sulfonic acid is chosen from alkyl or aryl sulfonic acids, more particularly aryl sulfonic acids such as benzenesulfonic acid, para-toluenesulfonic acid, naphthalenesulfonic acid, xylenesulfonic acid, cumenesulfonic acid, and mixtures thereof, and preferably chosen from cumenesulfonic acid and para-toluenesulfonic acid.

11. The process as claimed in any one of the preceding claims, **characterized in that** the mineral acid is chosen from sulfuric acid, hydrochloric acid, nitric acid, phosphoric acid and perchloric acid, and mixtures thereof, preferably chosen from sulfuric acid.

12. The process as claimed in any one of the preceding claims, **characterized in that** the stoichiometric excess between the reagents, namely between said compound comprising at least one tertiary hydroperoxide group and said compound containing at least one tertiary alcohol function, is between 0.01 and 1, preferably between 0.05 and 0.5 and more preferably between 0.1 and 0.3.

13. The process as claimed in any one of the preceding claims, **characterized in that** the step of placing in contact is performed at a temperature of between 10°C and 60°C, preferably between 20°C and 50°C.

14. The process as claimed in any one of the preceding claims, **characterized in that** it comprises a step prior to said step of placing in contact in the reaction medium, consisting of a step of mixing the mineral acid with the sulfonic acid outside said reaction medium.

15. The process as claimed in any one of the preceding claims, **characterized in that** the catalyst consists solely of the sulfonic acid and the mineral acid.
